# EUROPEAN PATENT SPECIFICATION

(11) **EP 3 888 531 B1**
(45) Date of publication and mention of the grant of the patent: **15.11.2023**
(21) Application number: 20167489.2
(22) Date of filing: 01.04.2020
(51) Int. Cl.: A61B 5/00

(54) **DEVICE AND METHOD FOR RASTER-SCAN OPTOACOUSTIC IMAGING**
VORRICHTUNG UND VERFAHREN ZUR OPTOAKUSTISCHEN RASTERABTASTUNG
DISPOSITIF ET PROCÉDÉ POUR IMAGERIE OPTO-ACOUSTIQUE À BALAYAGE RÉCURRENT

(43) Date of publication of application: 06.10.2021
(73) Proprietor: iThera Medical GmbH, 81379 München (DE)
(72) Inventor: Oakes, Keith, Daventry, Northamptonshire NN11 8EA (GB); Schwarz, Mathias, 81379 München (DE)
(74) Representative: Linsmeier, Josef

(56) References cited:
- WO-A1-2019/016361
- WO-A2-2012/080837
- SCHWARZ MATHIAS ET AL: "Unmixing chromophores in human skin with a 3D multispectral optoacoustic mesoscopy system", PROGRESS IN BIOMEDICAL OPTICS AND IMAGING, SPIE - INTERNATIONAL SOCIETY FOR OPTICAL ENGINEERING, BELLINGHAM, WA, US, vol. 9708, 15 March 2016 (2016-03-15), pages 970855-970855, XP060064699, ISSN: 1605-7422, DOI: 10.1117/12.2209333 ISBN: 978-1-5106-0027-0
- XP055532444
- Murad Omar ET AL: "Pushing the Optical Imaging Limits of Cancer with Multi-Frequency-Band Raster-Scan Optoacoustic Mesoscopy (RSOM)", NEOPLASIA, vol. 17, no. 2, 1 February 2015 (2015-02-01), pages 208-214, XP055518842, US ISSN: 1476-5586, DOI: 10.1016/j.neo.2014.12.010

## Description

The present invention relates to a device and corresponding method for raster-scan optoacoustic imaging according to the independent claims.

Raster-scan optoacoustic mesoscopy (RSOM) is an optoacoustic imaging technique, where an acoustic detector is scanned over a region of interest of an object, e.g. a biological tissue, emitting acoustic waves in response to an irradiation with a plurality of laser pulses. To ensure fast imaging and achieve motion-free image quality, laser pulse repetition rates of several hundreds of Hz to a few kHz are used. Currently, only single-wavelength solid-state lasers in the visible spectrum (e.g. 515 nm or 532 nm) achieve such high repetition rates with both sufficient energy and short pulse durations in the ns-range at relatively low cost.

Optoacoustic imaging enables multispectral excitation of the object under investigation, which allows for quantifying the concentrations of intrinsic chromophores (e.g. melanin, (de)oxyhemoglobin) or extrinsically administered absorbers. Typically, optical parametric oscillators (OPOs) are used to achieve multispectral excitation. However, OPOs are typically large and limited in their tuning speed, so that per-pulse tuning reaches the limits at a few hundred Hz.

SCHWARZ MATHIAS ET AL: "Unmixing chromophores in human skin with a 3D multispectral optoacoustic mesoscopy system", PROGRESS IN BIOMEDICAL OPTICS AND IMAGING, SPIE - INTERNATIONAL SOCIETY FOR OPTICAL ENGINEERING, BELLINGHAM, WA, US, vol. 9708, 15 March 2016 (2016-03-15), pages 970855-970855, discloses a multispectral raster-scan optoacoustic mesoscopy (MSOM) system in which tissue is excited by a flash-lamp pumped YAG/OPO (SpitLight 600 OPO, InnoLas Laser GmbH, Krailling, Germany) in the spectral range from 413 to 680 nm with a repetition rate of 50 Hz. The laser is operated in wavelength-sweep mode emitting consecutive pulses at various wavelengths that allows for automatic co-registration of multispectral datasets.

WO 2012/080837 A2 discloses a method for irradiating a medium, wherein an electromagnetic wave is modulated in frequency at a position of the medium. External modulators such as acousto-optic modulators (AOM) can be driven independently by clocks of frequencies which are adjusted such that the frequency difference between them is approximately equal to the frequency applied to an ultrasound system.

Mathias Schwarz, "Multispectral Optoacoustic Dermoscopy: Methods and Applications", PhD Thesis (2017), discloses implementations of raster-scan optoacoustic mesoscopy (RSOM), wherein light from a laser is coupled into a fiber bundle and directed onto a sample. The illumination fibers and an ultrasound transducer are raster-scanned in-tandem in the xy-plane by two piezoelectric stages. In some implementations a diode-pumped solid state (DPSS) laser is used, providing < 1 ns pulses at up to 2 kHz and an energy of up to 1 mJ per pulse at a wavelength of 532 nm. In another implementation the tissue is excited by a flash-lamp pumped yttrium aluminum garnet (YAG) optical parametric oscillator (OPO). An upgraded SpitLight OPO (InnoLas Laser GmbH, Krailling, Germany) emits 4 to 7 ns pulses in the spectral range from 413 to 2300 nm with a repetition rate of up to 100 Hz.

WO 2019/016361 A1 discloses a system and an according method for optoacoustic imaging, in particular for raster-scan optoacoustic mesoscopy. In some embodiments, a single-wavelength laser emitting pulses of 532 nm light lasting < 1 ns at repetition rates up to 2 kHz is used. In another embodiment, the tissue is excited using a tunable-wavelength OPO laser emitting 6 ns pulses in the spectral range from 420 to 2300 nm at a pulse repetition rate of 100 Hz.

Present invention is based on the problem to provide an improved device and method for multispectral raster-scan optoacoustic imaging.

The problem is solved by the device and method for multispectral raster-scan optoacoustic imaging according to the independent claims.

According to a first aspect of the invention, a device for raster-scan optoacoustic imaging comprises: a radiation source comprising at least one Raman laser source, the radiation source being configured to generate a plurality of pulses of electromagnetic radiation, each of the pulses comprising portions of electromagnetic radiation at two or more distinct wavelengths, and at least one acousto-optic tunable filter configured to select, from at least one of the pulses, one of the portions of electromagnetic radiation at one of the wavelengths; an irradiation unit configured to irradiate a region of interest of an object, in particular a biological tissue, with the selected portion of electromagnetic radiation at the one of the wavelengths of the at least one pulse; a detection unit configured to detect acoustic waves emitted from the region of interest in response to irradiating the region of interest with the selected portion of electromagnetic radiation at the one of the wavelengths of the at least one pulse; and a scanning unit configured to move the irradiation unit and detection unit, on the one hand, and the region of interest, on the other hand, along at least one dimension relative to each other so as to position the irradiation unit and detection unit at a plurality of different locations along the at least one dimension relative to the region of interest, and to control the detection unit to detect the acoustic waves at the plurality of locations.

According to a second aspect of the invention, a method for raster-scan optoacoustic imaging comprises the following steps: generating a plurality of pulses of electromagnetic radiation by means of a radiation source comprising at least one Raman laser source, each of the pulses comprising portions of electromagnetic radiation at two or more distinct wavelengths; selecting, from at least one of the pulses, one of the portions of electromagnetic radiation at one of the wavelengths by means of at least one acousto-optic tunable filter; irradiating a region of interest of an object, in particular a biological tissue, with the selected portion of electromagnetic radiation at the one of the wavelengths of the at least one pulse by means of an irradiation unit; detecting acoustic waves emitted from the region of interest in response to irradiating the region of interest with the selected portion of electromagnetic radiation at the one of the wavelengths of the at least one pulse by means of a detection unit; and moving the irradiation unit and detection unit, on the one hand, and the region of interest, on the other hand, along at least one dimension relative to each other so as to position the irradiation unit and detection unit at a plurality of different locations along the at least one dimension relative to the region of interest; and detecting acoustic waves at the plurality of locations.

Aspects of the invention are preferably based on the approach to provide at least one radiation source which comprises a Raman laser source and generates a plurality of pulses, each comprising two or more portions of electromagnetic radiation at two or more distinct wavelengths, and at least one acousto-optic tunable filter, in the following also referred to as "AOTF", which selects one of the portions of electromagnetic radiation at one of the wavelengths (also referred to as "selected wavelength") from, preferably each of, the pulses generated by the radiation source, and to irradiate the object with the selected portions of electromagnetic radiation at the respectively selected wavelengths.

Combining a radiation source comprising a Raman laser source with an AOTF which selects one of the portions of electromagnetic radiation at one of the wavelengths from the pulses generated by the radiation source allows for multispectral raster-scan optoacoustic imaging of an object with a high pulse repetition rate, in particular more than 1 kHz, a high pulse energy, in particular more than 10 µJ per pulse, a short pulse length, in particular less than 5 ns, and a "per-pulse tunability" of the wavelength, wherein each pulse, by which the object is irradiated, contains only selected electromagnetic radiation at the selected wavelength, i.e. at one of the two or more distinct wavelengths, and/or the selected wavelengths can be or are adjusted between successive laser pulses.

In summary, the invention allows for improved multispectral raster-scan optoacoustic imaging, in particular fast multispectral raster-scanning and yet motion-free images of high quality.

According to a preferred embodiment, the acousto-optic tunable filter is configured to select, from each of at least two of the pulses, in particular from each of at least two subsequent pulses, one of the portions of electromagnetic radiation at different wavelengths, the irradiation unit is configured to irradiate the region of interest with the selected portions of electromagnetic radiation at different wavelengths of the at least two, in particular subsequent, pulses and the scanning unit is configured to control the detection unit to detect acoustic waves emitted from the region of interest in response to irradiating the region of interest with the selected portions of electromagnetic radiation at different wavelengths of the at least two, in particular subsequent, pulses. In this way, the object can be irradiated with a series of pulses of electromagnetic radiation at different wavelengths.

In case that the pulses are subsequent pulses, the object can be irradiated with a series of subsequent pulses of electromagnetic radiation at different wavelengths, so that the time required for irradiating the object with electromagnetic radiation at a pre-defined set of two or more different wavelengths is minimized, because each of the subsequent pulses contains electromagnetic radiation at another wavelength.

Therefore, it is particularly preferred that the radiation source is configured to generate two or more subsequent pulses of electromagnetic radiation, each of the subsequent pulses comprising portions of electromagnetic radiation at the two or more distinct wavelengths, and the acousto-optic tunable filter is configured to select, from each of the two or more subsequent pulses, one of the portions of electromagnetic radiation at one of the wavelengths. In this way, a series of two or more subsequent pulses is obtained, wherein each of the pulses of the series contains a portion of electromagnetic radiation at one of the two or more distinct wavelengths. This is also referred to as "per-pulse tuning" in the narrower sense, wherein each pulse of a series of subsequent pulses contains electromagnetic radiation at another wavelength. Preferably, the irradiation unit is configured to irradiate the region of interest of the object with the series of the two or more subsequent pulses each of which containing a portion of electromagnetic radiation at one of the two or more distinct wavelengths, i.e. at another wavelength.

Preferably, the scanning unit is configured to control the detection unit to detect acoustic waves emitted from the region of interest in response to irradiating the region of interest with the series of the two or more subsequent pulses while continuously moving the irradiation unit and detection unit, on the one hand, and the region of interest, on the other hand, relative to each other.

Advantageously, due to per-pulse tuning the images obtained at the distinct wavelengths are only slightly or negligibly affected or "distorted", e.g. by a possible movement of the object during the (very short) period between two subsequent pulses, and therefore co-align exactly or virtually exactly. In contrast to this, if the images at the distinct wavelengths were recorded one after the other, i.e. one complete image at a certain wavelength after another image at another wavelength etc., the images at the distinct wavelengths would not be exactly co-aligned in case of a movement of the object during the (considerably longer) period between the acquisition of the different images.

The above embodiments shall be illustrated by means of the following example: The radiation source generates a plurality of pulses each comprising, e.g., a first, second, third and fourth portion of electromagnetic radiation at a first, second, third and fourth wavelength, e.g. at 532, 555, 579 and 606 nm. The AOTF selects, from a first pulse, the first portion of electromagnetic radiation at the first wavelength and the irradiation unit irradiates the region of interest of the object with the selected first portion of electromagnetic radiation at the first wavelength, e.g. 532 nm. Then, the AOTF selects, from an, in particular subsequent, second pulse the second portion of electromagnetic radiation at the second wavelength and the irradiation unit irradiates the region of interest of the object with the selected second portion of electromagnetic radiation at the second wavelength, e.g. 555 nm. Same applies accordingly for an, in particular subsequent, third pulse and an, in particular subsequent, fourth pulse. Of course, the example applies accordingly for less than four and more than four distinct wavelengths.

Preferably, the scanning unit is configured to control the detection unit to detect acoustic waves emitted from the region of interest in response to irradiating the region of interest with the selected portions of electromagnetic radiation at different wavelengths of the at least two, in particular subsequent, pulses while continuously moving the irradiation unit and detection unit, on the one hand, and the region of interest, on the other hand, relative to each other. According to this embodiment, the scanning motion of the irradiation and detection unit, on the one hand, and the object, on the other hand, relative to each other advantageously continues while the object is subsequently irradiated with pulses of electromagnetic radiation at the pre-defined different wavelengths, e.g. 2, 3, 4 or even more different wavelengths. Yet, because of the preferred high pulse repetition rate of in particular more than 1 kHz, which is enabled by the inventive combination of a radiation source including a Raman laser source and an AOTF, the time elapsing during irradiating the object with subsequent pulses at the different wavelengths is very short so that a possible motion of a living object, e.g. an organ or body part of a human or animal, during this time has no or only a negligible adverse effect on the image quality, so that in particular motion artifacts can be avoided or at least significantly reduced. For example, at a pulse repetition rate of approx. 1.4 kHz the time required for irradiating the object with four subsequent pulses at four different wavelengths is approx. 1/1.4 kHz x 4 = 0.7 ms x 4 = 2.8 ms which is considerably shorter than the time required for a typical physiological movement, e.g. approx. 0.8 s for heart beat.

Preferably, based on signals corresponding to the acoustic waves detected at the plurality of locations in response to irradiating the region of interest with the respectively selected portions of electromagnetic radiation at the different wavelengths, e.g. at three, four or five different wavelengths, contained in the pulses 2D or 3D images are reconstructed for each of the different wavelengths, e.g. three, four or five images at three, four or five, respectively, different wavelengths. Preferably, the signals and corresponding images are obtained using the above-described "per-pulse tuning" of the different wavelengths.

It is further preferred that the reconstructed images which were obtained for different wavelengths are spectrally processed, e.g. by performing spectral unmixing or other operations between images obtained at different wavelengths, e.g. division, subtraction, overlay etc.

In particular, spectral un-mixing requires a perfect co-alignment of the images at the different wavelengths which can be advantageously achieved with the device and method according to present disclosure. Preferably, due to per-pulse tuning all images at the different wavelengths are only slightly affected, e.g. by a possible movement of the object, in the same way, so that spectral processing remains robust.

In contrast to this, if images at different wavelength were recorded with devices and methods according to the prior art, i.e. one complete image at a certain wavelength after the other, the images at the different wavelengths would not be exactly co-aligned (e.g. due to possible movement of the object between the acquisition of the different images) so that spectral processing would fail.

Generally, the Raman laser source can be any type of laser in which the lightamplification mechanism is stimulated Raman scattering.

Preferably, the Raman laser source comprises a pump laser, in particular a solid-state laser, configured to emit a plurality of pulses of first radiation at a first wavelength. Moreover, the radiation source may further comprise at least one second harmonic generator (SHG) configured to convert a portion of the first radiation at the first wavelength to a second radiation at a second wavelength, wherein the second wavelength equals to half of the first wavelength. Preferably, the Raman laser source comprises an active medium, also referred to as "gain medium", in particular a glass fiber or a crystal or a gas cell, configured to emit, in response to a stimulation by the first radiation and/or second radiation, a plurality of pulses of Raman radiation, each of the pulses comprising one or more portions of electromagnetic radiation at one or more distinct Raman wavelengths. In other words, the Raman laser source is optically pumped, wherein pump photons of the first and/or second radiation are absorbed by the active medium and re-emitted as lower-frequency laser-light photons (so-called "Stokes" photons) by stimulated Raman scattering. The difference between the two photon energies, which is also referred to as "Stokes shift" or "Raman shift", is fixed and corresponds to a vibrational frequency of the gain medium. This allows for producing desired laser-output wavelengths by choosing the pump-laser wavelength appropriately.

Preferably, the active medium comprises Potassium Gadolinium Tungstate KGd(WO₄)₂, also referred to as KGW, preferably exhibiting a Stokes shift of 768 cm⁻¹ or 901 cm⁻¹, or Barium Nitrate Ba(NO₃)₂, preferably exhibiting a Stokes shift of 1048 cm⁻¹.

Preferably, the radiation source further comprises at least one extracting unit configured to extract a portion of the first radiation and/or a portion of the second radiation prior to stimulating the active medium so as to bypass the active medium and an alignment unit configured to co-align the extracted portion of the first radiation and/or second radiation with the Raman radiation.

Alternatively or additionally, pulses of Raman radiation emitted by the active medium comprise, in addition to the one or more portions of electromagnetic radiation at the one or more distinct Raman wavelengths, a residual portion of the first radiation at the first wavelength and/or a residual portion of the second radiation at the second wavelength and/or at least one residual portion of electromagnetic radiation (Raman radiation) at the one or more distinct Raman wavelengths. The residual portion of the first or second radiation preferably corresponds to a portion of the first or second radiation, respectively, which has not been converted to Raman radiation. Therefore, the respective residual portion is also referred to as "unconverted" first or second radiation. Same applies accordingly to the at least one residual portion of electromagnetic radiation (Raman radiation) at the one or more distinct Raman wavelengths, which in turn acts as a pump radiation in the active medium and has not been converted to Raman radiation at longer Raman wavelengths. Accordingly, the respective residual portion of electromagnetic radiation (Raman radiation) at the one or more distinct Raman wavelengths may also be referred to as "unconverted" Raman radiation.

Preferably, the residual portion of the first radiation at the first wavelength and/or the residual portion of the second radiation at the second wavelength and/or the at least one residual portion of electromagnetic radiation at the one or more distinct Raman wavelengths is, preferably inherently, co-aligned with the Raman radiation when exiting the Raman laser source, in particular the active medium.

Preferably, each of the plurality of pulses of electromagnetic radiation generated by the radiation source comprises Raman radiation at the one or more distinct Raman wavelengths and/or the extracted or residual portion of the first radiation at the first wavelength and/or the extracted or residual portion of the second radiation at the second wavelength. In this way, the "spectral content" of the pulses including the portions of electromagnetic radiation at the desired wavelengths can be provided in a simple and reliable way.

Preferably, the active medium exhibits different Raman shifts along different directions of propagation of the first radiation or of the second radiation within the active medium. In this way, the respectively desired Raman wavelength of the one or more portions of electromagnetic radiation contained in the Raman radiation can be selected in a simple and reliable manner by choosing the incidence angle at which the first and/or second radiation impinges on and/or enters the active medium.

Preferably, the active medium is a crystalline material exhibiting a crystallographic axis, and the polarization of the first radiation or of the second radiation is controllable and/or controlled so as to select a desired Raman shift by virtue of the orientation of said polarization relative to the crystallographic axis of the active medium. In this way, the respectively desired Raman wavelength of the one or more portions of electromagnetic radiation contained in the Raman radiation can be selected in a simple and reliable manner by choosing the orientation, in particular the angle, of the polarization of the first and/or second radiation relative to the crystallographic axis of the active medium.

Preferably, the polarization of the Raman radiation and/or of the first radiation and/or of the second radiation is controllable and/or controlled so as to align the Raman radiation and/or the extracted or residual portion of the first radiation and/or the extracted or residual portion of the second radiation to the at least one acousto-optic tunable filter to maximize the efficiency of the at least one acousto-optic tunable filter. For example, the acousto-optic tunable filter has an aperture which is defined by an ultrasound column with respect to which the polarization direction of the respective radiation is oriented. Preferably, the column orientation is also in a particular relationship to the geometry of the optic of the acousto-optic tunable filter, so that the polarization is implicitly also oriented with respect to the geometry of the optic of the acousto-optic tunable filter.

Preferably, controlling and/or providing a desired polarization or rotation of the polarization of the respective radiation can be achieved, e.g., by means of a polarization retardation plate, more specifically a so-called half-wave plate. Alternatively, the rotation can be accomplished using an active electro-optic element, such as a Pockels cell, by which the rotation can be accomplished at particularly high speed.

Preferably, the detection unit comprises a focused ultrasound transducer configured to detect the acoustic waves emitted from the region of interest. Preferably, the ultrasound transducer is a focused single-element ultrasound transducer with a central frequency in the range between 10 and 100 MHz and a bandwidth of more than 50 % of the central frequency.

Preferably, the irradiation unit is configured to irradiate the region of interest with a divergent beam of the selected portion of electromagnetic radiation of the at least one pulse. Preferably, the irradiation unit may comprise, e.g., a single fiber, multiple fibers, or fiber bundles, to illuminate the region of interest with the selected portions of electromagnetic radiation.

Further additional or alternative aspects, advantages, features and examples of the present invention will be apparent from the following description of following figures:
- Fig. 1: shows an example of a device for raster-scan optoacoustic imaging;
- Fig. 2: shows a first and second example of a radiation source;
- Fig. 3: shows a third and fourth example of a radiation source;
- Fig. 4: shows an example of an acousto-optic tunable filter; and
- Fig. 5: shows a table with examples of wavelengths of the electromagnetic radiation generated by the radiation source.

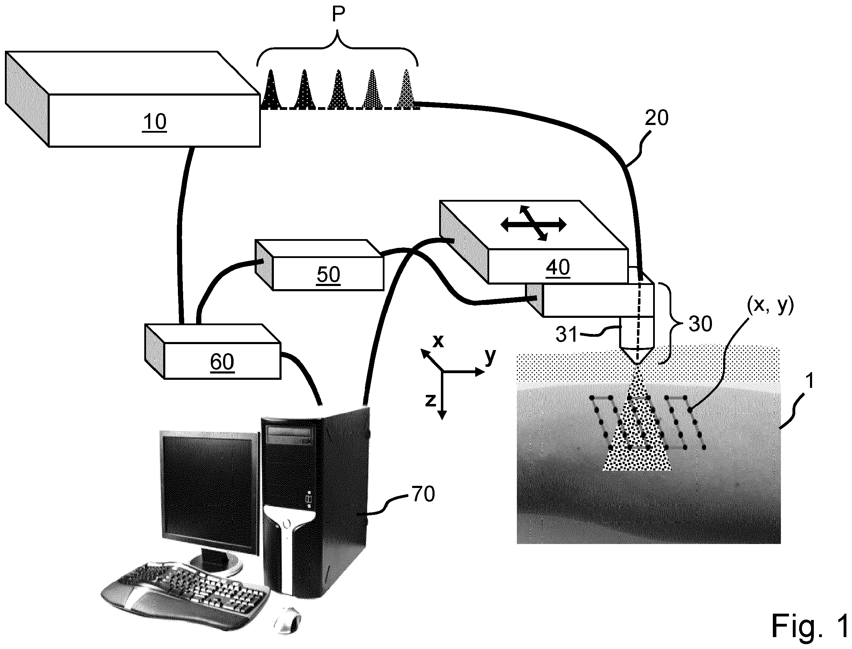

Figure 1 shows an example of a device for raster-scan optoacoustic imaging comprising a radiation source 10 which is configured to generate a plurality of pulses P of electromagnetic radiation at distinct wavelengths or wavelength ranges. In present example, the radiation source 10 is configured to generate sequences of five pulses P containing electromagnetic radiation at five distinct wavelengths.

The pulses P are coupled into a proximal end of an optical guiding element 20, e.g. a single fiber, multiple fibers or a fiber bundle, which is configured to guide the pulses P towards an object 1 to be imaged and to irradiate a region of interest of the object 1 with the pulses. In present example, the guiding element 20 is fed, as indicated by dashed lines, through a probe 30 such that a divergent beam of electromagnetic radiation of the pulses P emerging from a distal end of the guiding element 20 impinges on the object 1.

The probe 30 comprises a detection unit 31 which is configured to detect acoustic waves emitted from the region of interest of the object 1 in response to an irradiation with the pulses P. Preferably, the detection unit 31 comprises a focused single-element ultrasound transducer exhibiting a focus point and/or focal zone and being configured to detect ultrasound waves within a conically shaped sensitivity field of the transducer.

The probe 30 including the detection unit 31 and the distal end of the guiding element 20 is coupled to a scanning unit 40 which is configured to move the probe 30 relative to the object 1 in x- and y-direction to position the probe 30 at a plurality of different locations (x, y) in the x-y plane. In present example, the different locations (x, y) of the probe 30 relative to the object 1 are illustrated by a plurality of dots depicted over the region of interest of the object 1. Preferably, the dots constitute a grid of a plurality of positions (x, y) over which the detection unit 31, in particular the single-element transducer, is located, in particular centered, at the time when respective pulses P are generated and/or impinge on the region of interest. In present example, the probe 30 and/or detection unit 31 is moved relative to the object 1 along a meander-like or meandering trace. Optionally, the scanning unit 40 may be configured to move the probe 30 also in z-direction relative to the object 1.

Preferably, a control unit 70, for example a computer system, is provided for controlling the irradiation unit 10 and/or the scanning unit 40 and/or the detection unit 31 accordingly. Preferably, an amplifier 50 is provided for amplifying the signals corresponding to the acoustic waves detected by the detection unit 31. Preferably, a data acquisition card 60 (DAQ card) is provided via which the amplified signals are forwarded to the control unit 70 for processing and/or image reconstruction and/or multispectral processing (e.g. unmixing or other operations between images of different wavelength, e.g. division, subtraction, overlay, etc) and/or image display.

Preferably, the detection unit 31 detects acoustic waves emitted from the region of interest in response to subsequently irradiating the region of interest with the selected portions P of electromagnetic radiation at different wavelengths of subsequent pulses while continuously moving the probe 30 including the distal end of the irradiation unit 20 and the detection unit 31 relative to the object 1. That is, the scanning motion of the probe 30 relative to the object 1 advantageously continues while the object is subsequently irradiated with pulses of electromagnetic radiation at the selected different wavelengths, in present example five different wavelengths.

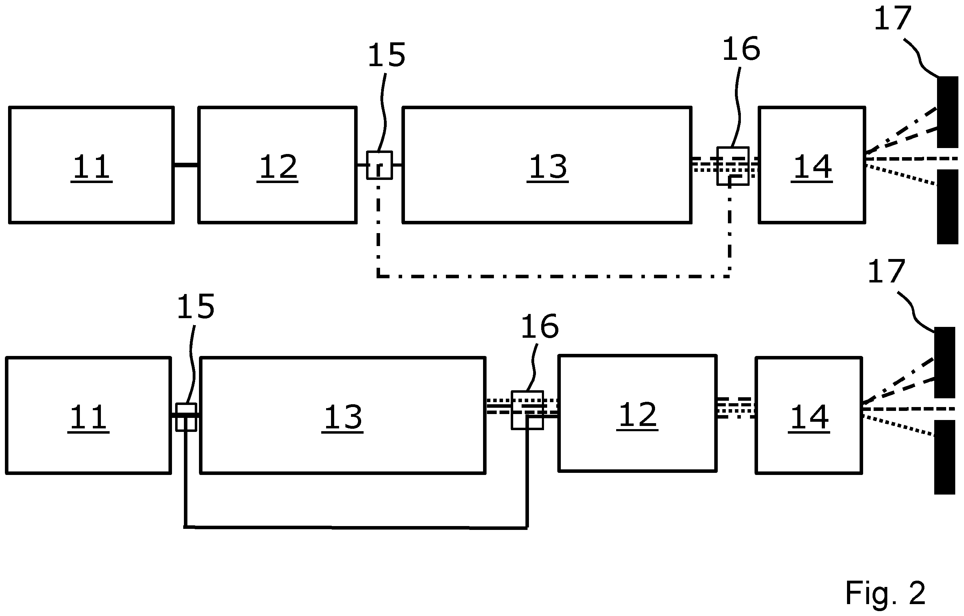

In particular when providing high pulse repetition rates of preferably more than 1 kHz, which are advantageously enabled by a radiation source 10 combining a Raman laser source and an acousto-optic tunable filter as will be explained in more detail below with reference to Figures 2 to 4, the time elapsing during irradiating the object 1 with subsequent pulses P at the different wavelengths is very short so that a possible motion of a living object, e.g. an organ or body part of a human or animal, during this time has no or only a negligible effect on the image quality, so that in particular motion artifacts can be avoided or at least significantly reduced. For example, at a pulse repetition rate of approx. 1.4 kHz the time required for irradiating the object with four subsequent pulses at four different wavelengths is approx. 1/1.4 kHz x 4 = 0.7 ms x 4 = 2.8 ms which is considerably shorter than the time required for a typical physiological movement, e.g. approx. 0.8 s for heart beat.

Figure 2 shows a first and second example of a radiation source, which is preferably used as the radiation source 10 in the device shown in Figure 1.

In the first example shown in the upper part of Figure 2, a pump laser 11, e.g. a solid-state laser, is provided to generate a plurality of pulses of first radiation at a first wavelength, e.g. at 1064 nm or 1030 nm. Further, a second harmonic generator 12 is provided which converts the first radiation at the first wavelength to a second radiation at a second wavelength, e.g. 532 nm or 515 nm, respectively, which equals half the first wavelength.

A first portion of the pulsed second radiation at the second wavelength is guided to an active medium 13, e.g. a glass fiber, crystal or gas cell, which emits, due to Raman scattering stimulated by the second radiation, a plurality of pulses of Raman radiation at different distinct wavelengths. In present example, as indicated by three differently dashed lines, pulsed Raman radiation at in total three different distinct wavelengths is generated.

In general, wavelengths of the Raman radiation (also referred to as Raman wavelengths) given in the context of present disclosure may vary by ±2 nm.

Preferably, an extracting unit 15 is provided which is configured to transmit the first portion of the pulsed second radiation at the second wavelength to the active medium 13 and to extract a second portion (indicated by dashed-dotted line) of the pulsed second radiation prior to reaching the active medium 13 so as to bypass the active medium 13 and to be aligned, preferably by means of an alignment unit 16, with the pulsed Raman radiation emerging from the active medium 13. In this way, in present example, a plurality of pulses of electromagnetic radiation each comprising in total four portions of electromagnetic radiation at four different wavelengths, e.g. 532 nm (bypassed second radiation) and 555 nm, 579 nm and 606 nm (Raman radiation), is obtained.

Further, an acousto-optic filter 14 is provided which is configured to select from each of the obtained pulses of electromagnetic radiation one out of four portions of electromagnetic radiation at one of the four distinct wavelengths.

Preferably, the acousto-optic filter 14 is configured to deflect the different portions of electromagnetic radiation depending on the respective wavelength and to be controlled to deflect the portions of electromagnetic radiation such that only a pre-defined portion (i.e. the portion to be selected) passes an aperture 17, while the other portions are blocked.

Preferably, the acousto-optic filter 14 is configured to deflect portions of electromagnetic radiation at different wavelengths so quickly that the different portions contained in subsequent pulses can be subsequently selected, which is also referred as "per-pulse tuning" in connection with present disclosure. In this way, in present example, a first portion of electromagnetic radiation at a first wavelength, e.g. 532 nm, of a first pulse passes the aperture 17, a second portion of electromagnetic radiation at a second wavelength, e.g. 555 nm, of a subsequent second pulse passes the aperture 17, and so on.

In the second example shown in the lower part of Figure 2, a first portion of a plurality of pulses of first radiation at a first wavelength, e.g. at 1064 nm or 1030 nm, generated by pump laser 11 is transmitted to the active medium 13 which generates a plurality of pulses of Raman radiation at distinct wavelengths, e.g. at in total three different distinct wavelengths (as indicated by three differently dashed lines).

Preferably, an extracting unit 15 is provided which is configured to transmit the first portion of the pulsed first radiation at the first wavelength to the active medium 13 and to extract a second portion of the pulsed first radiation prior to reaching the active medium 13 so as to bypass the active medium 13 and to be aligned, preferably by means of alignment unit 16, with the pulsed Raman radiation emerging from the active medium 13. In this way, in present example, a plurality of pulses of electromagnetic radiation each comprising in total four portions of electromagnetic radiation at four different wavelengths, e.g. 1064 nm (bypassed first radiation) and e.g. 1159 nm, 1272 nm and 1410 nm (Raman radiation), is obtained.

The second harmonic generator 12 converts the portions of electromagnetic radiation at the different (initial) wavelengths contained in each pulse to portions of electromagnetic radiation at different wavelengths corresponding to respectively half the (initial) wavelengths, e.g. 532 nm, 579 nm, 636 nm and 705 nm, respectively.

From each of the pulses of electromagnetic radiation obtained in this way, one out of the four portions of electromagnetic radiation at one of the four distinct wavelengths is selected by means of acousto-optic filter 14. The above explanations regarding the opto-acoustic filter 14 and the aperture 17 apply accordingly.

For further details on the second example of the radiation source, the above explanations given with reference to the first example of the radiation source apply accordingly.

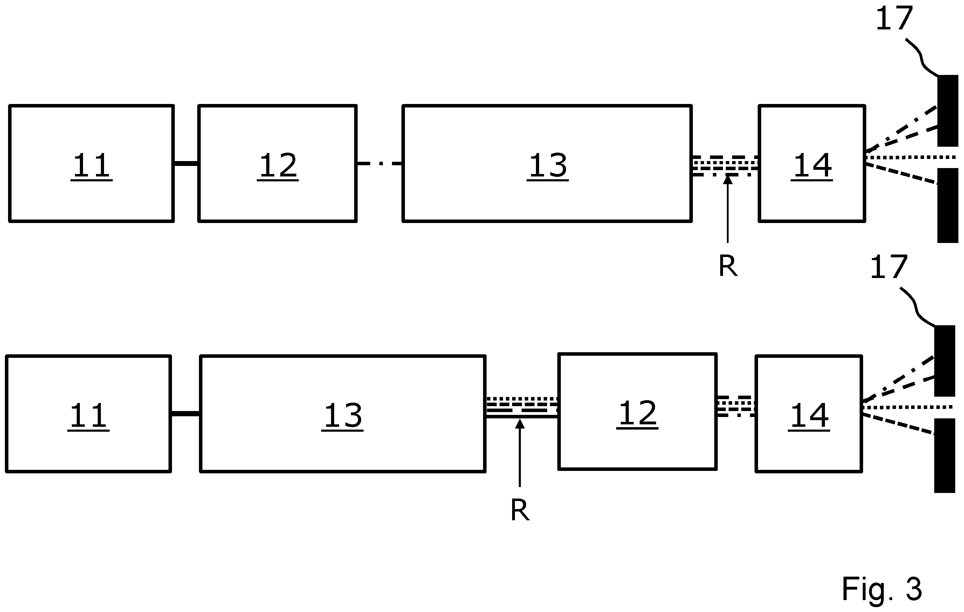

Figure 3 shows a third and fourth example of a radiation source, which is preferably used as the radiation source 10 in the device shown in Figure 1. Basically, the above explanations with reference to the first and second example of the radiation source apply accordingly to the third and fourth example, respectively.

In contrast to the first and second example, however, no extraction unit 15 and/or bypassing is used to combine a portion of pulsed pump radiation (i.e. second portion of pulsed second radiation in the first example, second portion of pulsed first radiation in the second example) with the portions pulsed Raman radiation emerging from the active medium 13.

Rather, in the third example, from the second radiation (indicated by dashed dotted line) emerging from the second harmonic generator 12 and coupled into the active medium 13, a residual portion R is not converted into Raman radiation and emerges from the active medium 13. The residual portion R is intrinsically co-aligned with the different portions of stimulated Raman radiation (indicated by differently dashed lines) so that an additional alignment unit 16 (see Figure 2) is dispensable. The above explanations regarding the opto-acoustic filter 14 and the aperture 17 apply accordingly.

Likewise, in the fourth example, from the first radiation (indicated by solid line) emerging from the pump laser 11 and coupled into the active medium 13, a residual portion R is not converted into Raman radiation and emerges from the active medium 13 in a co-aligned manner with the different portions of stimulated Raman radiation (indicated by differently dashed lines) so that an additional alignment unit 16 (see Figure 2) is dispensable. The above explanations regarding the second harmonic generator 12, the opto-acoustic filter 14 and the aperture 17 apply accordingly.

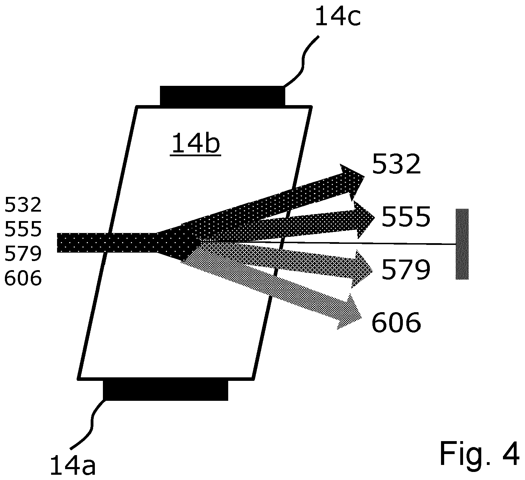

Figure 4 shows an example of an acousto-optic tunable filter, which is preferably used as the acousto-optic filter 14 in the radiation source shown in Figures 2 and 3.

Preferably, the opto-acoustic filter, which may also be referred to as acousto-optic modulator (AOM) or Bragg cell, uses the acousto-optic effect to diffract the pulsed electromagnetic radiation containing several portions at different wavelengths (in present example 532 nm, 555 nm, 579 nm and 606 nm) using sound waves generated by a piezoelectric transducer 14a attached to a material 14b such as quartz or glass. An acoustic absorber 14c is provided at a side of the material 14b opposite to the transducer 14a. An oscillating electric signal drives the transducer 14c to vibrate, which creates sound waves in the material 14b which act as moving periodic planes of expansion and compression that change the index of refraction of the material 14b. The incoming electromagnetic radiation scatters off the resulting periodic index modulation and interference occurs so that the portions of electromagnetic radiation at different wavelengths are split up as exemplarily shown in the Figure.

Further preferred or alternative aspects of present disclosure are discussed in the following.

The disclosed device and method provide a fast tuning multispectral RSOM scanner using a Raman laser source in combination with an acousto-optic tunable filter (AOTF), which reaches pulse repetition rates of more than 1 kHz and a per-pulse tunability of the wavelength. This allows for fast multispectral raster scanning with intrinsic co-registration of the 3D volume between different wavelengths.

Preferably, the preferably focused single-element ultrasound transducer has a central frequency in the range from 10 to 100 MHz and/or a bandwidth of more than 50 % of the respective central frequency.

Preferably, 3D images can be reconstructed based on the acoustic waves detected separately for each wavelength.

Further spectral processing is preferred to visualize the imaged absorbers, using spectral information from the different wavelengths.

Preferably, the pump laser 11 is a solid state laser, e.g. 1064 nm (Nd:YAG) or 1030 nm (Yb:YAG), with a pulse length in the range from 200 ps to 10 ns.

Preferably, the active material 13 provides a set of Raman wavelengths in the visible range, e.g. but not limited to 555 nm, 579 nm, 606 nm, 635 nm. Preferably, the set of Raman wavelengths is chosen depending on the orientation of the polarization relative to the medium (crystal).

Preferably, the set of Raman wavelengths is fine-tuned by the pump wavelength, Raman active material, polarization, and second harmonic generation to get wavelengths that fit well to the spectra of absorbers to be distinguished, e.g. 532-555-579-606 nm to discriminate between melanin, oxyhemoglobin, deoxyhemoglobin.

Preferably, the tuning speed of the AOTF can reach more than 1 kHz. Due to non-perfect filtering of the AOTF, residual energies from the other wavelengths may be present. Preferably, the remaining wavelengths get damped by output aperture 17. Preferably, the set of wavelengths gets continuously repeated during the scan.

Preferably, spectral processing scheme of the different wavelength images is performed using at least one of the following methods:
- Linear regression making use of known absorption spectra, e.g. spectra of melanin, oxyhemoglobin, deoxyhemoglobin, etc.
- ICA, in particular guided ICA
- Ratios
- Addition or subtraction
- Color/Channel mixing.

Preferably, present disclosure relates to a multispectral raster-scanning optoacoustic imaging device and method, which uses laser pulses of different wavelengths to excite optical absorbers within a sample, and a single element focused ultrasound detector to capture the ultrasound signal emitted by the exited absorbers following energy conversion due to the photoacoustic effect.

According to a preferred implementation, the irradiation unit, in particular the distal end of optical guiding element 20, is co-axially arranged with an axis of the detection unit 31 and/or the probe 30 and guided through the detection unit 31 and/or probe 30 to enable a co-axial irradiation and detection.

Preferably, the Raman laser medium 13 is pumped by a solid-state laser, e.g. with 1064nm or 1030nm, with a pulse length of shorter than 10 ns, preferably shorter than 5 ns.

Preferably, second harmonic generation can be performed either directly on the pump wavelength or after the Raman active medium 13 (crystal). A portion of the pump wavelength or frequency-doubled pump wavelength may be extracted and co-aligned after the Raman medium 13, while the major energy passes through the Raman medium 13 producing a set of Stokes-shifted wavelengths depending on the pump wavelength. By choosing the polarization of the pump beam different sets of Raman wavelengths can be selected (linear or rotate polarization).

Preferably, at the laser outlet an acousto-optic-tunable filter is provided to filter out one of the Raman wavelengths and/or the frequency-doubled pump wavelength.

Preferably, the set of Raman wavelengths is chosen depending on the orientation of the polarization relative to the active medium 13 (crystal). Preferably, the wavelengths 555 nm, 579 nm and 606 nm originate from the rotate polarization.

Preferably, the pulse length of pump and Raman radiation is in the range of 2 to 3 ns. All of the Raman wavelengths are intrinsically co-aligned. In addition, a fraction of the pump wavelength can be extracted before the Raman crystal, and co-aligned with the Raman wavelengths before the AOTF.

Preferably, an AOTF driver is used to generate a sequence of signals, preferably electric signals provided to the piezoelectric transducer 14a at the medium 14b of the AOTF, to filter out a single wavelength at the laser output, while the rest of the wavelengths is damped inside the laser housing.

Due to not-perfect filtering of the AOTF (side-lobes), residual portions from neighboring wavelengths are present in addition to (main) portions at the different wavelengths. Preferably, this may be considered in the unmixing scheme.

In the following, examples of approximate main and residual portions at different wavelengths are given:
- R1: 97% 532 nm + 3% 555 nm
- R2: 94% 555 nm + 3% 532 nm + 3% 579 nm
- R3: 85% 579 nm + 9% 555 nm + 6% 606 nm
- R4: 94% 606 nm + 6% 579 nm

Preferably, the laser energies are calibrated before unmixing procedures are performed.

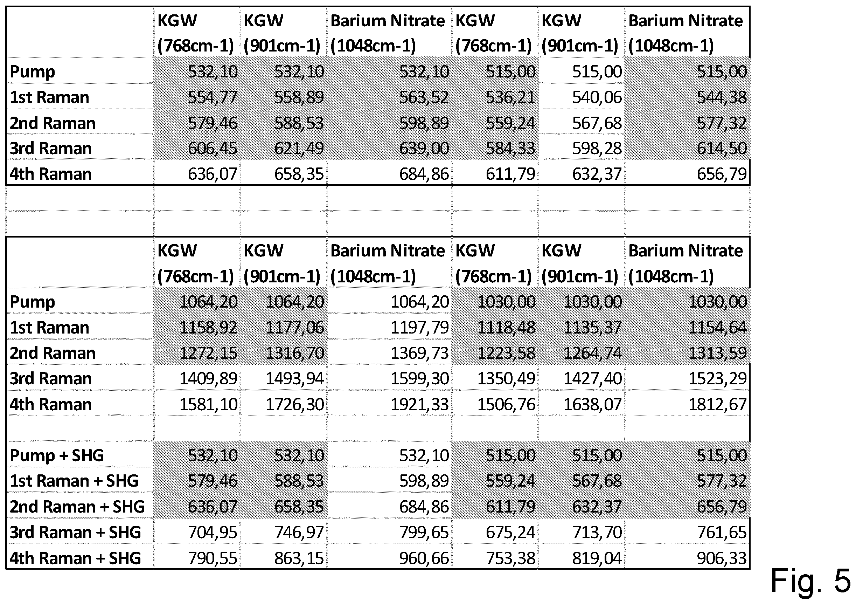

Figure 5 shows a table with examples of wavelengths (in units of [nm]) of electromagnetic radiation generated by a radiation source 10 comprising Potassium Gadolinium Tungstate KGd(WO₄)₂, also referred to as KGW, exhibiting a Stokes shift of 768 cm⁻¹ or 901 cm⁻¹, or Barium Nitrate Ba(NO₃)₂ exhibiting a Stokes shift of 1048 cm⁻¹ as the active medium 13 (see Figures 2 and 3) for different pump wavelengths ("Pump").

In the upper part of the table, the pump wavelengths of 532 nm and 515 nm correspond to second wavelengths of second radiation within the meaning of present disclosure and were obtained by converting first radiation generated by pump laser 11 at 1064 nm or 1030 nm, respectively, with a second harmonic generator 12 (see upper part of Figure 2 and 3 and respective description). Dependent on the pump wavelength and the material of the active medium 13 of the Raman source, Raman radiation at different Raman wavelengths "1^{st} Raman" to "4^{th} Raman" is obtained.

In the lower part of the table, the pump wavelengths of 1064 nm and 1030 nm correspond to first wavelengths of first radiation within the meaning of present disclosure which was generated by pump laser 11 at 1064 nm or 1030 nm, respectively. Dependent on the pump wavelength and the material of the active medium 13 of the Raman source, Raman radiation at different Raman wavelengths "1^{st} Raman" to "4^{th} Raman" is obtained. In case of a subsequent conversion of the obtained radiation (Pump and Raman) with a second harmonic generator 12 (see lower part of Figure 2 and 3 and respective description), radiation at half the wavelength "Pump + SHG" and "1^{st} Raman + SHG" to "4^{th} Raman + SHG" is obtained in each case.

In the table, preferably used sets of distinct wavelengths ("Pump", "Pump + SHG", "Raman", "Raman + SHG") comprising four (upper part of table) or, respectively, three (lower part of table) distinct wavelengths are highlighted by a shaded background. The highlighted sets of distinct wavelengths are particularly advantageous for obtaining multispectral raster-scan optoacoustic images with high informative and/or diagnostic value.

## Claims

1. A device for raster-scan optoacoustic imaging, the device comprising:
- a radiation source (10) comprising at least one Raman laser source (11, 13), the radiation source (10) being configured to generate a plurality of pulses of electromagnetic radiation, each of the pulses comprising portions of electromagnetic radiation at two or more distinct wavelengths, and at least one acousto-optic tunable filter (14) configured to select, from at least one of the pulses, one of the portions (P) of electromagnetic radiation at one of the wavelengths,
- an irradiation unit (20) configured to irradiate a region of interest of an object (1), in particular a biological tissue, with the selected portion (P) of electromagnetic radiation of the at least one pulse,
- a detection unit (31) configured to detect acoustic waves emitted from the region of interest in response to irradiating the region of interest with the selected portion (P) of electromagnetic radiation of the at least one pulse, and
- a scanning unit (40) configured to move the irradiation unit (20) and detection unit (31), on the one hand, and/or the region of interest, on the other hand, along at least one dimension (x, y) relative to each other so as to position the irradiation unit (20) and detection unit (31) at a plurality of different locations along the at least one dimension (x, y) relative to the region of interest, and to control the detection unit (31) to detect the acoustic waves at the plurality of locations.

2. The device according to claim 1,
- the acousto-optic tunable filter (14) being configured to select, from each of at least two of the pulses, in particular from each of at least two subsequent pulses, one of the portions (P) of electromagnetic radiation at different wavelengths,
- the irradiation unit (20) being configured to irradiate the region of interest with the selected portions (P) of electromagnetic radiation of the at least two, in particular subsequent, pulses and
- the scanning unit (40) being configured to control the detection unit (31) to detect acoustic waves emitted from the region of interest in response to irradiating the region of interest with the selected portions (P) of electromagnetic radiation of the at least two, in particular subsequent, pulses.

3. The device according to claim 2, the scanning unit (40) being configured to control the detection unit (31) to detect acoustic waves emitted from the region of interest in response to irradiating the region of interest with the selected portions (P) of electromagnetic radiation of the at least two, in particular subsequent, pulses while continuously moving the irradiation unit (20) and detection unit (31), on the one hand, and the region of interest, on the other hand, relative to each other.

4. The device according to any preceding claim,
- the Raman laser source (11, 13) comprising a pump laser (11), in particular a solid-state laser, configured to emit a plurality of pulses of first radiation at a first wavelength and/or
- the radiation source (10) further comprising at least one second harmonic generator (12) configured to convert a portion of the first radiation at the first wavelength to a second radiation at a second wavelength, wherein the second wavelength equals to half of the first wavelength.

5. The device according to claim 4, the Raman laser source (11, 13) comprising an active medium (13), in particular a glass fiber or a crystal or a gas cell, configured to emit, in response to a stimulation by the first radiation and/or second radiation, a plurality of pulses of Raman radiation, each of the pulses comprising one or more portions of electromagnetic radiation at one or more distinct Raman wavelengths.

6. The device according to claim 5, the radiation source (10) further comprising
- at least one extracting unit (15) configured to extract a portion of the first radiation and/or a portion of the second radiation prior to stimulating the active medium (13) so as to bypass the active medium (13) and
- an alignment unit (16) configured to co-align the extracted portion of the first radiation and/or second radiation with the Raman radiation.

7. The device according to claim 5 or 6, each of the pulses of Raman radiation emitted by the active medium (13) comprising, in addition to the one or more portions of electromagnetic radiation at the one or more distinct Raman wavelengths, a residual portion (R) of the first radiation at the first wavelength and/or a residual portion of the second radiation at the second wavelength and/or at least one residual portion of electromagnetic radiation at the one or more distinct Raman wavelengths.

8. The device according to claim 7, the residual portion (R) of the first radiation at the first wavelength and/or the residual portion of the second radiation at the second wavelength and/or the at least one residual portion of electromagnetic radiation at the one or more distinct Raman wavelengths being co-aligned with the Raman radiation.

9. The device according to any of the claims 5 to 8, wherein each of the pulses (P) of the plurality of pulses of electromagnetic radiation generated by the radiation source (10) comprises Raman radiation at the one or more distinct Raman wavelengths.

10. The device according to claim 9, wherein each of the pulses of the plurality of pulses (P) of electromagnetic radiation generated by the radiation source (10) further comprises the extracted or residual portion of the first radiation at the first wavelength and/or the extracted or residual portion of the second radiation at the second wavelength.

11. The device according to any of the claims 5 to 10, wherein the active medium (13) exhibits different Raman shifts along different directions of propagation of the first radiation or of the second radiation within the active medium (13).

12. The device according to claim 11, wherein the active medium (13) is a crystalline material exhibiting a crystallographic axis and the polarization of the first radiation or of the second radiation is controllable and/or controlled so as to select a desired Raman shift by virtue of the orientation of said polarization relative to the crystallographic axis of the active medium (13).

13. The device according to any of the claims 5 to 12, wherein the polarization of the Raman radiation and/or of the first radiation and/or of the second radiation is controllable and/or controlled so as to align the Raman radiation and/or the extracted or residual portion of the first radiation and/or the extracted or residual portion of the second radiation to the at least one acousto-optic tunable filter (14) to maximize the efficiency of the at least one acousto-optic tunable filter (13).

14. The device according to any preceding claim, wherein
- the detection unit (31) comprises a focused ultrasound transducer configured to detect the acoustic waves emitted from the region of interest and/or
the irradiation unit (20) is configured to irradiate the region of interest with a divergent beam of the selected portion (P) of electromagnetic radiation of the at least one pulse.

15. A method for raster-scan optoacoustic imaging comprising the following steps:
- generating a plurality of pulses of electromagnetic radiation by means of a radiation source (10) comprising at least one Raman laser source (11, 13), each of the pulses comprising portions of electromagnetic radiation at two or more distinct wavelengths,
- selecting, from at least one of the pulses, one of the portions (P) of electromagnetic radiation at one of the wavelengths by means of at least one acousto-optic tunable filter (AOTF),
- irradiating a region of interest of an object (1), in particular a biological tissue, with the selected portion (P) of electromagnetic radiation of the at least one pulse by means of an irradiation unit,
- detecting acoustic waves emitted from the region of interest in response to irradiating the region of interest with the selected portion (P) of electromagnetic radiation of the at least one pulse by means of a detection unit (31), and
- moving the irradiation unit (20) and detection unit (31), on the one hand, and/or the region of interest, on the other hand, along at least one dimension (x, y) relative to each other so as to position the irradiation unit (20) and detection unit (31) at a plurality of different locations along the at least one dimension (x, y) relative to the region of interest, and
- detecting acoustic waves at the plurality of locations.

## Patentansprüche

1. Vorrichtung zur optoakustischen Rasterscan-Bildgebung, wobei die Vorrichtung aufweist:
- eine Strahlungsquelle (10), die mindestens eine Raman-Laserquelle (11, 13) aufweist, wobei die Strahlungsquelle (10) so konfiguriert ist, dass sie eine Vielzahl von Pulsen elektromagnetischer Strahlung erzeugt, wobei jeder der Pulse Anteile elektromagnetischer Strahlung bei zwei oder mehr verschiedenen Wellenlängen aufweist, und mindestens ein akusto-optisch abstimmbares Filter (14) aufweist, das so konfiguriert ist, dass es aus mindestens einem der Pulse einen der Anteile (P) elektromagnetischer Strahlung bei einer der Wellenlängen auswählt,
- eine Bestrahlungseinheit (20), die so konfiguriert ist, dass sie einen interessierenden Bereich eines Objekts (1), insbesondere ein biologisches Gewebe, mit dem ausgewählten Anteil (P) der elektromagnetischen Strahlung des mindestens einen Pulses bestrahlt,
- eine Detektionseinheit (31), die so konfiguriert ist, dass sie akustische Wellen detektiert, die von dem interessierenden Bereich als Reaktion auf die Bestrahlung des interessierenden Bereichs mit dem ausgewählten Anteil (P) der elektromagnetischen Strahlung des mindestens einen Pulses emittiert werden, und
- eine Abtasteinheit (40), die so konfiguriert ist, dass sie die Bestrahlungseinheit (20) und die Detektionseinheit (31) einerseits und/oder den interessierenden Bereich andererseits entlang mindestens einer Dimension (x, y) relativ zueinander bewegt, um die Bestrahlungseinheit (20) und die Detektionseinheit (31) an einer Vielzahl von verschiedenen Orten entlang der mindestens einen Dimension (x, y) relativ zu dem interessierenden Bereich zu positionieren, und die Detektionseinheit (31) steuert, um die akustischen Wellen an der Vielzahl von Orten zu erfassen.

2. Vorrichtung nach Anspruch 1, wobei
- das akusto-optisch abstimmbare Filter (14) dazu ausgebildet ist, aus jedem der mindestens zwei, insbesondere aus jedem der mindestens zwei aufeinanderfolgenden, Pulse einen der Anteile (P) elektromagnetischer Strahlung mit unterschiedlichen Wellenlängen auszuwählen,
- die Bestrahlungseinheit (20) so konfiguriert ist, dass sie den interessierenden Bereich mit den ausgewählten Anteilen (P) der elektromagnetischen Strahlung der mindestens zwei, insbesondere aufeinanderfolgenden, Pulse bestrahlt, und
- die Abtasteinheit (40) so konfiguriert ist, dass sie die Detektionseinheit (31) so steuert, dass sie akustische Wellen erfasst, die von dem interessierenden Bereich als Reaktion auf die Bestrahlung des interessierenden Bereichs mit den ausgewählten Anteilen (P) der elektromagnetischen Strahlung der mindestens zwei, insbesondere aufeinanderfolgenden, Pulse emittiert werden.

3. Vorrichtung nach Anspruch 2, wobei die Abtasteinheit (40) so konfiguriert ist, dass sie die Detektionseinheit (31) steuert, um akustische Wellen zu detektieren, die von dem interessierenden Bereich als Reaktion auf die Bestrahlung des interessierenden Bereichs mit den ausgewählten Anteilen (P) der elektromagnetischen Strahlung der mindestens zwei, insbesondere aufeinanderfolgenden, Pulse ausgesendet werden, während die Bestrahlungseinheit (20) und die Detektionseinheit (31) einerseits und der interessierende Bereich andererseits kontinuierlich relativ zueinander bewegt werden.

4. Vorrichtung nach einem der vorhergehenden Ansprüche, wobei
- die Raman-Laserquelle (11, 13) einen Pumplaser (11), insbesondere einen Festkörperlaser, aufweist, der so konfiguriert ist, dass er eine Vielzahl von Pulsen einer ersten Strahlung mit einer ersten Wellenlänge emittiert, und/oder
- die Strahlungsquelle (10) ferner mindestens einen Generator für eine zweite Harmonische (12) aufweist, der so konfiguriert ist, dass er einen Anteil der ersten Strahlung mit der ersten Wellenlänge in eine zweite Strahlung mit einer zweiten Wellenlänge umwandelt, wobei die zweite Wellenlänge der Hälfte der ersten Wellenlänge entspricht.

5. Vorrichtung nach Anspruch 4, wobei die Raman-Laserquelle (11, 13) ein aktives Medium (13), insbesondere eine Glasfaser oder einen Kristall oder eine Gaszelle, aufweist, das so konfiguriert ist, dass es als Reaktion auf eine Stimulation durch die erste Strahlung und/oder die zweite Strahlung eine Vielzahl von Pulsen von Raman-Strahlung emittiert, wobei jeder der Pulse einen oder mehrere Anteile elektromagnetischer Strahlung bei einer oder mehreren unterschiedlichen Raman-Wellenlängen aufweist.

6. Vorrichtung nach Anspruch 5, wobei die Strahlungsquelle (10) ferner aufweist
- mindestens eine Extraktionseinheit (15), die so konfiguriert ist, dass sie einen Anteil der ersten Strahlung und/oder einen Anteil der zweiten Strahlung vor der Stimulation des aktiven Mediums (13) extrahiert, um das aktive Medium (13) zu umgehen, und
- eine Ausrichtungseinheit (16), die so konfiguriert ist, dass sie den extrahierten Anteil der ersten Strahlung und/oder der zweiten Strahlung mit der Raman-Strahlung ausrichtet.

7. Vorrichtung nach Anspruch 5 oder 6, wobei jeder der vom aktiven Medium (13) emittierten Raman-Strahlungspulse zusätzlich zu dem einen oder den mehreren Anteilen der elektromagnetischen Strahlung bei der einen oder den mehreren unterschiedlichen Raman-Wellenlängen einen Restanteil (R) der ersten Strahlung bei der ersten Wellenlänge und/oder einen Restanteil der zweiten Strahlung bei der zweiten Wellenlänge und/oder mindestens einen Restanteil der elektromagnetischen Strahlung bei der einen oder den mehreren unterschiedlichen Raman-Wellenlängen aufweist.

8. Vorrichtung nach Anspruch 7, wobei der Restanteil (R) der ersten Strahlung bei der ersten Wellenlänge und/oder der Restanteil der zweiten Strahlung bei der zweiten Wellenlänge und/oder der mindestens eine Restanteil der elektromagnetischen Strahlung bei der einen oder den mehreren unterschiedlichen Raman-Wellenlängen mit der Raman-Strahlung ausgerichtet ist.

9. Vorrichtung nach einem der Ansprüche 5 bis 8, wobei jeder der Pulse (P) der von der Strahlungsquelle (10) erzeugten Vielzahl von Pulsen elektromagnetischer Strahlung Raman-Strahlung bei einer oder mehreren unterschiedlichen Raman-Wellenlängen aufweist.

10. Vorrichtung nach Anspruch 9, wobei jeder der Pulse der von der Strahlungsquelle (10) erzeugten Vielzahl von Pulsen (P) elektromagnetischer Strahlung ferner den extrahierten oder restlichen Anteil der ersten Strahlung mit der ersten Wellenlänge und/oder den extrahierten oder restlichen Anteil der zweiten Strahlung mit der zweiten Wellenlänge aufweist.

11. Vorrichtung nach einem der Ansprüche 5 bis 10, wobei das aktive Medium (13) unterschiedliche Raman-Verschiebungen entlang unterschiedlicher Ausbreitungsrichtungen der ersten Strahlung oder der zweiten Strahlung innerhalb des aktiven Mediums (13) aufweist.

12. Vorrichtung nach Anspruch 11, wobei das aktive Medium (13) ein kristallines Material ist, das eine kristallographische Achse aufweist, und die Polarisation der ersten Strahlung oder der zweiten Strahlung steuerbar ist und/oder so gesteuert wird, dass eine gewünschte Raman-Verschiebung aufgrund der Orientierung der Polarisation relativ zur kristallographischen Achse des aktiven Mediums (13) gewählt wird.

13. Vorrichtung nach einem der Ansprüche 5 bis 12, wobei die Polarisation der Raman-Strahlung und/oder der ersten Strahlung und/oder der zweiten Strahlung steuerbar ist und/oder so gesteuert wird, dass die Raman-Strahlung und/oder der extrahierte oder verbleibende Anteil der ersten Strahlung und/oder der extrahierte oder verbleibende Anteil der zweiten Strahlung auf den mindestens einen akusto-optisch abstimmbaren Filter (14) ausgerichtet wird, um die Effizienz des mindestens einen akusto-optisch abstimmbaren Filters (14) zu maximieren.

14. Vorrichtung nach einem der vorhergehenden Ansprüche, wobei
- die Detektionseinheit (31) einen fokussierten Ultraschallwandler aufweist, der so konfiguriert ist, dass er die von dem interessierenden Bereich emittierten akustischen Wellen detektiert, und/oder
die Bestrahlungseinheit (20) so konfiguriert ist, dass sie den interessierenden Bereich mit einem divergenten Strahl des ausgewählten Anteils (P) der elektromagnetischen Strahlung des mindestens einen Pulses bestrahlt.

15. Verfahren zur optoakustischen Rasterscan-Bildgebung, das die folgenden Schritte aufweist:
- Erzeugen einer Vielzahl von Pulsen elektromagnetischer Strahlung mittels einer Strahlungsquelle (10), die mindestens eine Raman-Laserquelle (11, 13) aufweist, wobei jeder der Pulse Anteile elektromagnetischer Strahlung bei zwei oder mehr verschiedenen Wellenlängen aufweist,
- Auswählen, aus mindestens einem der Pulse, eines der Anteile (P) der elektromagnetischen Strahlung bei einer der Wellenlängen mittels mindestens eines akusto-optisch abstimmbaren Filters (AOTF),
- Bestrahlen eines interessierenden Bereichs eines Objekts (1), insbesondere eines biologischen Gewebes, mit dem ausgewählten Anteil (P) der elektromagnetischen Strahlung des mindestens einen Pulses mittels einer Bestrahlungseinheit,
- Detektieren von akustischen Wellen, die von dem interessierenden Bereich als Reaktion auf die Bestrahlung des interessierenden Bereichs mit dem ausgewählten Anteil (P) der elektromagnetischen Strahlung des mindestens einen Pulses mittels einer Detektionseinheit (31) emittiert werden, und
- Bewegen der Bestrahlungseinheit (20) und der Detektionseinheit (31) einerseits und/oder des interessierenden Bereichs andererseits entlang mindestens einer Dimension (x, y) relativ zueinander, um die Bestrahlungseinheit (20) und die Detektionseinheit (31) an einer Vielzahl von verschiedenen Orten entlang der mindestens einen Dimension (x, y) relativ zu dem interessierenden Bereich zu positionieren, und
- Erfassen von akustischen Wellen an der Vielzahl von Orten.

## Revendications

1. Dispositif d'imagerie optoacoustique à balayage de trame, le dispositif comprenant :
- une source de rayonnement (10) comprenant au moins une source laser Raman (11, 13), la source de rayonnement (10) étant configurée pour générer une pluralité d'impulsions de rayonnement électromagnétique, chacune des impulsions comprenant des parties de rayonnement électromagnétique à deux longueurs d'onde distinctes ou plus, et au moins un filtre accordable acousto-optique (14) configuré pour sélectionner, parmi au moins une des impulsions, une des parties (P) de rayonnement électromagnétique à l'une des longueurs d'onde,
- une unité de rayonnement (20) configurée pour exposer une région d'intérêt d'un objet (1), en particulier un tissu biologique, à la partie sélectionnée (P) de rayonnement électromagnétique de l'au moins une impulsion,
- une unité de détection (31) configurée pour détecter des ondes acoustiques émises par la région d'intérêt en réponse à l'exposition de la région d'intérêt à la partie sélectionnée (P) de rayonnement électromagnétique de l'au moins une impulsion, et
- une unité de balayage (40) configurée pour déplacer l'unité de rayonnement (20) et l'unité de détection (31), d'une part, et/ou la région d'intérêt, d'autre part, le long d'au moins une dimension (x, y) les unes par rapport aux autres de manière à positionner l'unité de rayonnement (20) et l'unité de détection (31) en une pluralité d'emplacements différents le long de l'au moins une dimension (x, y) par rapport à la région d'intérêt, et pour commander l'unité de détection (31) pour détecter les ondes acoustiques à la pluralité d'emplacements.

2. Dispositif selon la revendication 1,
- le filtre accordable acousto-optique (14) étant configuré pour sélectionner, parmi chacune d'au moins deux des impulsions, en particulier parmi chacune d'au moins deux impulsions successives, une des parties (P) de rayonnement électromagnétique à différentes longueurs d'onde,
- l'unité de rayonnement (20) étant configurée pour exposer la région d'intérêt aux parties sélectionnées (P) de rayonnement électromagnétique des au moins deux impulsions, en particulier successives, et
- l'unité de balayage (40) étant configurée pour commander l'unité de détection (31) pour détecter des ondes acoustiques émises par la région d'intérêt en réponse à l'exposition de la région d'intérêt aux parties sélectionnées (P) de rayonnement électromagnétique des au moins deux impulsions, en particulier successives.

3. Dispositif selon la revendication 2, l'unité de balayage (40) étant configurée pour commander l'unité de détection (31) pour détecter des ondes acoustiques émises par la région d'intérêt en réponse à l'exposition de la région d'intérêt aux parties sélectionnées (P) de rayonnement électromagnétique des au moins deux impulsions, en particulier successives, tout en déplaçant en continu l'unité de rayonnement (20) et l'unité de détection (31), d'une part, et la région d'intérêt, d'autre part, les unes par rapport aux autres.

4. Dispositif selon une quelconque revendication précédente,
- la source laser Raman (11, 13) comprenant un laser de pompage (11), en particulier un laser à solide, configuré pour émettre une pluralité d'impulsions de premier rayonnement à une première longueur d'onde et/ou
- la source de rayonnement (10) comprenant en outre au moins un deuxième générateur d'harmoniques (12) configuré pour convertir une partie du premier rayonnement à la première longueur d'onde en un deuxième rayonnement à une deuxième longueur d'onde, dans lequel la deuxième longueur d'onde équivaut à la moitié de la première longueur d'onde.

5. Dispositif selon la revendication 4, la source laser Raman (11, 13) comprenant un milieu actif (13), en particulier une fibre de verre ou un cristal ou une pile à gaz, configuré pour émettre, en réponse à une stimulation par le premier rayonnement et/ou le deuxième rayonnement, une pluralité d'impulsions de rayonnement Raman, chacune des impulsions comprenant une ou plusieurs parties de rayonnement électromagnétique à une ou plusieurs longueurs d'onde Raman distinctes.

6. Dispositif selon la revendication 5, la source de rayonnement (10) comprenant en outre
- au moins une unité d'extraction (15) configurée pour extraire une partie du premier rayonnement et/ou une partie du deuxième rayonnement avant de stimuler le milieu actif (13) de manière à contourner le milieu actif (13) et
- une unité d'alignement (16) configurée pour coaligner la partie extraite du premier rayonnement et/ou du deuxième rayonnement sur le rayonnement Raman.

7. Dispositif selon la revendication 5 ou 6, chacune des impulsions de rayonnement Raman émises par le milieu actif (13) comprenant, en plus des une ou plusieurs parties de rayonnement électromagnétique aux une ou plusieurs longueurs d'onde Raman distinctes, une partie résiduelle (R) du premier rayonnement à la première longueur d'onde et/ou une partie résiduelle du deuxième rayonnement à la deuxième longueur d'onde et/ou au moins une partie résiduelle de rayonnement électromagnétique aux une ou plusieurs longueurs d'onde Raman distinctes.

8. Dispositif selon la revendication 7, la partie résiduelle (R) du premier rayonnement à la première longueur d'onde et/ou la partie résiduelle du deuxième rayonnement à la deuxième longueur d'onde et/ou l'au moins une partie résiduelle de rayonnement électromagnétique aux une ou plusieurs longueurs d'onde Raman distinctes étant coalignées sur le rayonnement Raman.

9. Dispositif selon l'une quelconque des revendications 5 à 8, dans lequel chacune des impulsions (P) de la pluralité d'impulsions de rayonnement électromagnétique généré par la source de rayonnement (10) comprend un rayonnement Raman aux une ou plusieurs longueurs d'onde Raman distinctes.

10. Dispositif selon la revendication 9, dans lequel chacune des impulsions de la pluralité d'impulsions (P) de rayonnement électromagnétique généré par la source de rayonnement (10) comprend en outre la partie extraite ou résiduelle du premier rayonnement à la première longueur d'onde et/ou la partie extraite ou résiduelle du deuxième rayonnement à la deuxième longueur d'onde.

11. Dispositif selon l'une quelconque des revendications 5 à 10, dans lequel le milieu actif (13) présente différents décalages Raman le long de différentes directions de propagation du premier rayonnement ou du deuxième rayonnement à l'intérieur du milieu actif (13).

12. Dispositif selon la revendication 11, dans lequel le milieu actif (13) est un matériau cristallin présentant un axe cristallographique et la polarisation du premier rayonnement ou du deuxième rayonnement est régulable et/ou régulée de manière à sélectionner un décalage Raman souhaité en raison de l'orientation de ladite polarisation par rapport à l'axe cristallographique du milieu actif (13).

13. Dispositif selon l'une quelconque des revendications 5 à 12, dans lequel la polarisation du rayonnement Raman et/ou du premier rayonnement et/ou du deuxième rayonnement est régulable et/ou régulée de manière à aligner le rayonnement Raman et/ou la partie extraite ou résiduelle du premier rayonnement et/ou la partie extraite ou résiduelle du deuxième rayonnement par rapport à l'au moins un filtre accordable acousto-optique (14) pour augmenter au maximum l'efficacité de l'au moins un filtre accordable acousto-optique (14).

14. Dispositif selon une quelconque revendication précédente, dans lequel
- l'unité de détection (31) comprend un transducteur à ultrasons focalisés configuré pour détecter les ondes acoustiques émises par la région d'intérêt et/ou
- l'unité de rayonnement (20) est configurée pour exposer la région d'intérêt à un faisceau divergent de la partie sélectionnée (P) de rayonnement électromagnétique de l'au moins une impulsion.

15. Procédé d'imagerie optoacoustique à balayage de trame comprenant les étapes suivantes :
- la génération d'une pluralité d'impulsions de rayonnement électromagnétique au moyen d'une source de rayonnement (10) comprenant au moins une source laser Raman (11, 13), chacune des impulsions comprenant des parties de rayonnement électromagnétique à deux longueurs d'onde distinctes ou plus,
- la sélection, parmi au moins l'une des impulsions, d'une des parties (P) de rayonnement électromagnétique à l'une des longueurs d'onde au moyen d'au moins un filtre accordable acousto-optique (AOTF),
- l'exposition d'une région d'intérêt d'un objet (1), en particulier un tissu biologique, à la partie sélectionnée (P) de rayonnement électromagnétique de l'au moins une impulsion au moyen d'une unité de rayonnement,
- la détection d'ondes acoustiques émises par la région d'intérêt en réponse à l'exposition de la région d'intérêt à la partie sélectionnée (P) de rayonnement électromagnétique de l'au moins une impulsion au moyen d'une unité de détection (31), et
- le déplacement de l'unité de rayonnement (20) et de l'unité de détection (31), d'une part, et/ou de la région d'intérêt, d'autre part, le long d'au moins une dimension (x, y) les unes par rapport aux autres de manière à positionner l'unité de rayonnement (20) et l'unité de détection (31) en une pluralité d'emplacements différents le long de l'au moins une dimension (x, y) par rapport à la région d'intérêt, et
- la détection d'ondes acoustiques à la pluralité d'emplacements.
